# EUROPEAN PATENT SPECIFICATION

(11) **EP 2 114 875 B1**
(45) Date of publication and mention of the grant of the patent: **15.08.2012**
(21) Application number: 08713853.3
(22) Date of filing: 21.01.2008
(51) Int. Cl.: C07C 405/00, C07D 333/16, A61K 31/381

(54) **SUBSTITUTED ARYLCYLOPENTENES AS THERAPEUTIC AGENTS**
SUBSTITUIERTE ARYLCYCLOPENTENE ALS THERAPEUTIKA
ARYLCYCLOPENTENES SUBSTITUES COMME AGENTS THERAPEUTIQUES

(30) Priority: 22.01.2007 US 886018 P
(43) Date of publication of application: 11.11.2009
(73) Proprietor: ALLERGAN, INC., Irvine CA 92612 (US)
(72) Inventor: OLD, David, W., Irvine, CA 92620 (US); NGO, Vinh, X., Huntington Beach, CA 92646 (US)
(74) Representative: HOFFMANN EITLE
(86) International application number: PCT/US2008/051540
(87) International publication number: WO 2008/091810

(56) References cited:
- WO-A-03/040126
- WO-A-2006/076370

## Description

### DESCRIPTION OF THE INVENTION

Ocular hypotensive agents are useful in the treatment of a number of various ocular hypertensive conditions, such as post-surgical and post-laser trabeculectomy ocular hypertensive episodes, glaucoma, and as presurgical adjuncts.

Glaucoma is a disease of the eye characterized by increased intraocular pressure. On the basis of its etiology, glaucoma has been classified as primary or secondary. For example, primary glaucoma in adults (congenital glaucoma) may be either open-angle or acute or chronic angle-closure. Secondary glaucoma results from pre-existing ocular diseases such as uveitis, intraocular tumor or an enlarged cataract.

The underlying causes of primary glaucoma are not yet known. The increased intraocular tension is due to the obstruction of aqueous humor outflow. In chronic open-angle glaucoma, the anterior chamber and its anatomic structures appear normal, but drainage of the aqueous humor is impeded. In acute or chronic angle-closure glaucoma, the anterior chamber is shallow, the filtration angle is narrowed, and the iris may obstruct the trabecular meshwork at the entrance of the canal of Schlemm. Dilation of the pupil may push the root of the iris forward against the angle, and may produce pupilary block and thus precipitate an acute attack. Eyes with narrow anterior chamber angles are predisposed to acute angle-closure glaucoma attacks of various degrees of severity.

Secondary glaucoma is caused by any interference with the flow of aqueous humor from the posterior chamber into the anterior chamber and subsequently, into the canal of Schlemm. Inflammatory disease of the anterior segment may prevent aqueous escape by causing complete posterior synechia in iris bombe, and may plug the drainage channel with exudates. Other common causes are intraocular tumors, enlarged cataracts, central retinal vein occlusion, trauma to the eye, operative procedures and intraocular hemorrhage.

Considering all types together, glaucoma occurs in about 2% of all persons over the age of 40 and may be asymptotic for years before progressing to rapid loss of vision. In cases where surgery is not indicated, topical β-adrenoreceptor antagonists have traditionally been the drugs of choice for treating glaucoma.

Certain eicosanoids and their derivatives are currently commercially available for use in glaucoma management. Eicosanoids and derivatives include numerous biologically important compounds such as prostaglandins and their derivatives. Prostaglandins can be described as derivatives of prostanoic acid which have the following structural formula:

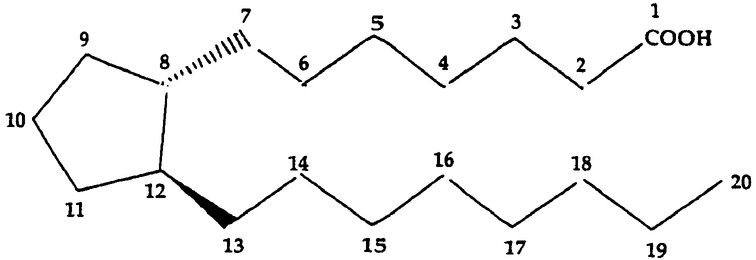

Various types of prostaglandins are known, depending on the structure and substituents carried on the alicyclic ring of the prostanoic acid skeleton. Further classification is based on the number of unsaturated bonds in the side chain indicated by numerical subscripts after the generic type of prostaglandin [e.g. prostaglandin E₁(PGE₁). prostaglandin E₂(PGE₂)], and on the configuration of the substituents on the alicyclic ring indicated by a or β[e.g. prostaglandin F_{2α}(PGF_{2β})].

An embodiment of the present invention is a compound represented by the following formula or a pharmaceutically-acceptable salt thereof: 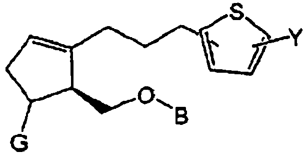 wherein:
Y is an organic acid functional group, an amide or ester of an organic acid functional group comprising up to 14 carbon atoms, hydroxymethyl, an ether of hydroxymethyl comprising up to 14 carbon atoms, or a tetrazolyl functional group;
G is H or OH; and
B is an optionally-substituted aryl group or an optionally-substituted heteroaryl group, the total number of non-hydrogen atoms in the optional substituents being 20 or less.

This compound is useful for treating glaucoma or ocular hypertension.

"Bioisosteres are substituents or groups that have chemical or physical similarities, and which produce broadly similar biological properties." Silverman, Richard B., The Organic Chemistry of Drug Design and Drug Action, 2nd Edition, Amsterdam: Elsevier Academic Press, 2004, p. 29.

Organic acid functional groups are bioisoteres of carboxylic acids. An organic acid functional group is an addic functional group on an organic molecule. Organic acid functional groups may comprise an oxide of carbon, sulfur, or phosphorous. Thus in certain compounds Y is a carboxylic acid, sulfonic acid, or phosphonic acid functional group.

Additionally, an amide or ester of one of the organic acids mentioned above comprising up to 14 carbon atoms is also contemplated for Y. In an ester, a hydrocarbyl moiety replaces a hydrogen atom of an acid such as in a carboxylic acid ester, e.g. CO₂Me, CO₂Et, etc.

In an amide, an amine group replaces an OH of the acid. Examples of amides include CON(R²)₂, CON(OR²)R², CON(CH₂CH₂OH)₂, and CONH(CH₂CH₂OH) where R² is independently H, C₁-C₆ alkyl, phenyl, or biphenyl. Moieties such as CONHSO₂R² are also amides of the carboxylic acid notwithstanding the fact that they may also be considered to be amides of the sulfonic acid R²-SO₃H. The following amides are also specifically contemplated, CONSO₂-biphenyl, CONSO₂-phenyl, CONSO₂-heteroaryl, and CONSO₂-naphthyl. The biphenyl, phenyl, heteroaryl, or naphthyl may be substituted or unsubstituted.

Han et. al. (Biorganic & Medicinal Chemistry Letters 15 (2005) 3487-3490) has recently shown that the groups shown below are suitable bioisosteres for a carboxylic acid. The activity of compounds with these groups in inhibiting HCV NS3 protease was comparable to or superior to similar compounds where the group is replaced by CO₂H. Thus, Y could be any group depicted below.

Carboxylic acid bioisosteres according to Han et. *al.*

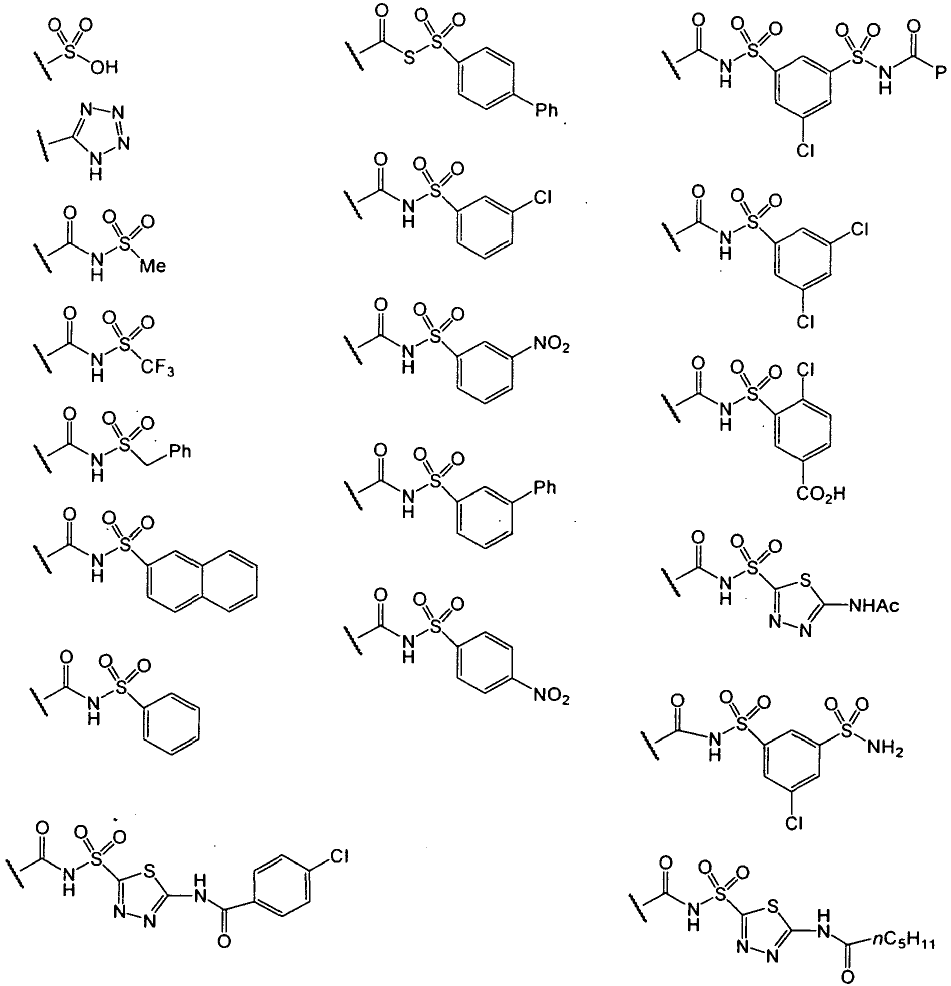

Y may also be hydroxymethyl or an ether thereof comprising up to 14 carbon atoms. An ether is a functional group wherein a hydrogen of an hydroxyl is replaced by carbon, e.g., Y is CH₂OCH₃, CH₂OCH₂CH₃, etc. These groups are also bioisosteres of a carboxylic acid.

"Up to 14 carbon atoms" means that the entire Y moiety, including the carbonyl carbon of a carboxylic acid ester or amide, and both carbon atoms in the -CH₂O-C of an ether has 0, 1, 2, 3, 4, 5, 6, 7, 8, 9, 10, 11, 12, 13, or 14 carbon atoms.

Finally Y may be a tetrazolyl functional group.

Thus the structures below exemplify what is meant by tetrazolyl; carboxylic acid, phosphonic acid, sulfonic acid, and their esters and amides; hydroxymethyl and ether of hydroxymethyl. In these structures, R is H or hydrocarbyl, subject to the constraints defined herein.

Each structure below represents a specific embodiment which is individually contemplated, as well as pharmaceutical acceptable salts of compounds which are represented by the structures.

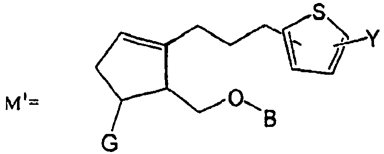

| **Organic Acids** | **Esters** | **Amides** |
|---|---|---|
| M¹-CO₂H | M¹-CO₂R | M¹-CO₂NR₂ |
| Carboxylic Acid | Carboxylic Acid Ester | Carboxylic Acid Amide |
| M¹-P(O)(OH)₂ | M¹-P(O)(OH)OR | M¹-P(O)(OH)NR₂ |
| Phosphonic Acid | Phosphonic Acid Ester | Phosphonic Acid Amide |
| M¹-SO₃H | M¹-SO₃R | M¹-SO₃NR₂ |
| Sulfonic Acid | Sulfonic Acid Ester | Sulfonic Acid Amide |
| M¹-CH₂OH | M¹-CH₂OR | 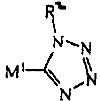 |
| Hydroxymethyl | Ether | Tetrazolyl |

A tetrazolyl functional group is another bioisostere of a carboxylic acid. An unsubstituted tetrazolyl functional group has two tautomeric forms, which can rapidly interconvert in aqueous or biological media, and are thus equivalent to one another. These tautomers are shown below.

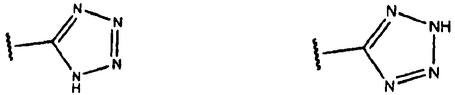

Additionally, if R² is C₁-C₆ alkyl, phenyl, or biphenyl, other isomeric forms of the tetrazolyl functional group such as the one shown below are also possible, unsubstituted and hydrocarbyl substituted tetrazolyl up to C₁₂ are considered to be within the scope of the term "tetrazolyl."

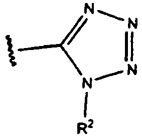

In one embodiment, Y is CO₂R², CON(R²)₂, CON(OR²)R², CON(CH₂CH₂OH)₂, CONH(CH₂CH₂OH), CH₂OH, P(O)(OH)₂, CONHSCO₂R², SO₂N(R²)₂, SO₂NHR²,

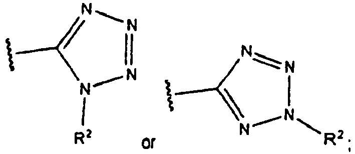

wherein R² is independently H, C₁-C₆ alkyl, unsubstituted phenyl, or unsubstituted biphenyl.

In one embodiment the thiophene-containing substituent is 5-(3-propyl)thiophen-2-yl.

G is H or OH. Thus, compounds according to one of the following structures are possible.

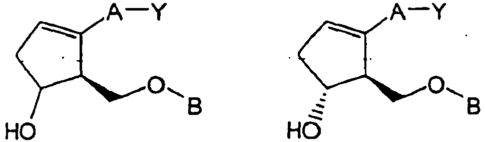

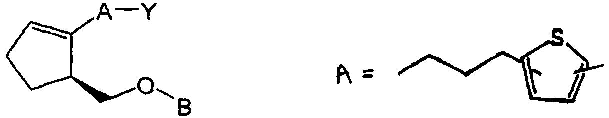

B is aryl or heteroaryl.

Aryl is an aromatic ring or ring system such as phenyl, naphthyl, biphenyl, and the like.

Heteroaryl is aryl having one or more N, O, or S atoms in the ring, i.e. one or more ring carbons are substituted by N, O, and/or S. While not intending to be limiting, examples of heteroaryl include thienyl, pyridinyl, furyl, benzothienyl, benzofuryl, imidizololyl, indolyl, and the like.

A substituent of aryl or heteroaryl should be stable and may have up to 20 non-hydrogen atoms each and as many hydrogen atoms as necessary, wherein the non-hydrogen atoms are C, N. O, S, P, F, Cl, Br, and/or I in any stable combination. However, the total number of non-hydrogen atoms on all of the substituents combined must also be 20 or less. In addition to the atoms listed above, a substituent may also have a metal cation or other stable cation having an atom not listed above if the substituent is acidic and the salt form is stable. For example, -OH may form an -O-Na⁺ salt or CO₂H may form a CO₂⁻K⁺ salt. Any cation of the salt is not counted in the 20 non-hydrogen atoms. Thus a substituent may be:

hydrocarbyl, i.e. a moiety consisting of only carbon and hydrogen such as alkyl, alkenyl, alkynyl, and the like, including linear, branched or cyclic hydrocarbyl, and combinations thereof;

hydrocarbyloxy, meaning O-hydrocarbyl such as OCH₃, OCH₂CH₃, O-cyclohexyl, etc, up to 19 carbon atoms;

other ether substituents such as CH₂OCH₃, (CH₂)₂OCH(CH₃)₂,

thioether substituents including S-hydrocarbyl and other thioether substituents;

hydroxyhydrocarbyl, meaning hydrocarbyl-OH, including hydroxyalkyl, such as CH₂OH, C(CH₃)₂OH, up to 19 carbon atoms;

nitrogen substituents such as NO₂, CN, including

amino, such as NH₂, NH(CH₂CH₃OH), NHCH₃;

carbonyl substituents, such as CO₂H, ester, amide;

halogen, such as chloro, fluoro, bromo

fluorocarbyl, such as CF₃, CF₂CF₃, etc.;

phosphorous substituents, such as PO₃²⁻;

sulfur substituents, including S-hydrocarbyl, SH, SO₃H, SO₂-hydrocarbyl, SO₃-hydrocarbyl

Substituted aryl or heteroaryl may have as many substituents as the ring or ring system will bear, and the substituents may be the same or different. Thus, for example, an aryl ring or a heteroaryl ring may be substituted with chloro and methyl; methyl, OH, and F; CN, NO₂, and ethyl; including any conceivable substituent or combination of substituent possible in light of this disclosure.

Subsituted aryl or substituted heteroaryl also includes a bicyclic or polycyclic ring system wherein one or more rings are aromatic and one or more rings are not. For example, indanonyl, indanyl, indanolyl, tetralonyl, are substituted aryl and are also substituted phenyl. For this type of polycyclic ring system, an aromatic or heteroaromatic ring, not a non-aromatic ring, must be attached to the remainder of the molecule, i.e. the part of the molecule that is not B. In other words, in any structure depicting -B herein, where - is a bond, the bond is a direct bond to an aromatic ring.

Hydrocarbyl is a moiety consisting of carbon and hydrogen, including,
1. alkyl, which is hydrocarbyl containing no double or triple carbon-carbon bonds; alkyl includes
   - linear alkyl, cyclic alkyl, branched alkyl, and combinations thereof;
   - C₁₋₃ alkyl, which refers to alkyl having 1, 2, or 3 carbon atoms, including methyl, ethyl, isopropyl, cyclopropyl, n-propyl, and the like;
   - C₁₋₆ alkyl, which refers to alkyl having 1, 2, 3, 4, 5, or 6 carbon atoms; including, methyl, ethyl, propyl isomers, cyclopropyl, butyl isomers, cyclobutyl, pentyl isomers, cyclopentyl, hexyl isomers, cyclohexyl;
   - combinations of these terms are possible, and their meanings should be obvious to those of ordinary skill in the art; for example C₁₋₆ linear alkyl would refer to C₁₋₆ alkyl which is also linear;
2. alkenyl, which is hydrocarbyl containing one or more carbon-carbon double bonds; alkenyl includes
   - linear alkenyl, cyclic alkenyl, branched alkenyl, and combinations thereof;
   - alkenyl having 1, 2, 3, or more carbon-carbon double bonds;
3. alkynyl, which is hydrocarbyl containing one or more carbon-carbon triple bonds; akynyl includes :
   - linear alkynyl, cyclic alkynyl, branched alkynyl, and combinations thereof;
   - alkynyl having 1, 2, 3, or more carbon-carbon double bonds;
4. aryl, provided that it contains no heteroatoms either in a ring or as a substituent; and
5. combinations of any of the above;

C₁₋₆ hydroxylalkyl is hydroxyalkyl having 1, 2, 3, 4, 5, or 6 carbon atoms.

In another embodiment, B is substituted or unsubstituted phenyl.

In another embodiment, B is substituted or unsubstituted thienyl.

In another embodiment, B is substituted or unsubstituted naphthyl.

In another embodiment, B is substituted or unsubstituted furyl.

In another embodiment, B is substituted or unsubstituted pyridinyl.

In another embodiment, B is substituted or unsubstituted benzothienyl.

In another embodiment, B is substituted or unsubstituted indanyl.

In another embodiment, B is substituted or unsubstituted tetralonyl.

In another embodiment, B has 1, 2, 3, 4, or 5 substituents, wherein each substituent has one or more carbon, fluorine, chlorine, bromine, oxygen, sulfur, or atoms; and wherein all substituents taken together consist of 0, 1, 2, 3, 4, 5, 6, 7, 8, 9 or 10 carbon atoms; 0, 1, 2, 3, 4, 5, 6, 7, 8 or 9 fluorine atoms; 0, 1, 2 or 3 chlorine atoms, 0, 1, 2 or 3 bromine atoms, 0, 1, 2 or 3 oxygen atoms; 0,1, 2, or 3 sulfur atoms; 0, 1, 2, or 3 nitrogen atoms.

In another embodiment, B has 1, 2, 3, 4, or 5 substituents, wherein each substituent has one or more carbon, fluorine, chlorine, bromine, or oxygen atoms; and wherein all substituents taken together consist of 0, 1, 2, 3, 4, 5, 6, 7, 8, 9 or 10 carbon atoms; 0, 1, 2, 3, 4, 5, 6, 7, 8 or 9 fluorine atoms; 0, 1, 2 or 3 chlorine atoms, 0, 1, 2 or 3 bromine atoms, and 0, 1, 2 or 3 oxygen atoms.

In another embodiment, B has a substituent of the formula CₐH_{b}O_{c}; wherein a is 0, 1, 2, 3, 4, 5, 6, 7, 8 or 9, b is 0, 1, 2, 3, 4, 5, 6, 7, 8, 9, 10, 11, 12, 13, 14, 15, 16, 17, 18 or 19; and c is 0, 1, 2, or 3.

In another embodiment, B has 1, 2, 3, or 4 alkyl substituents having 1, 2, 3, 4, 5, 6, 7, 8, 9 or 10 carbon atoms.

In another embodiment, B has a hydroxyalkyl substituent having 0, 1, 2, 3, 4, 5, 6, 7, 8, 9 or 10 carbon atoms and 1 or 2 hydroxy moieties.

In another embodiment, B has an alkyl substituent having 0, 1, 2, 3, 4, 5, 6, 7, 8, 9 or 10 carbon atoms.

In another embodiment, B has 1, 2, 3, or 4 halogen substituents.

In another embodiment, B has 1, 2, 3, or 4 chloro subsituents.

In another embodiment, B has 1 chloro substituent.

In another embodiment, B has 2 chloro substituents.

In another embodiment, B has 1, 2, 3, or 4 trifluoromethyl substituents.

In another embodiment, B has 1, 2, or 3 trifluoromethyl substituents.

In another embodiment, B has 1 trifluoromethyl substituent.

In another embodiment, B has 2 trifluoromethyl substituents.

In another embodiment, B has a hydroxyl substituent.

Examples of useful moieties for B are depicted below. Each is individually contemplated as an embodiment.

| | | | |
|---|---|---|---|
| Structure: | 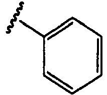 | 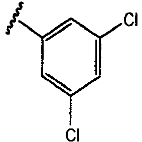 | 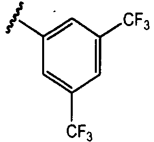 |
| Name: | unsubstituted phenyl | 3,5-dichlorophenyl | 3,5-di(trifluoromethyl)phenyl |
| Structure: | 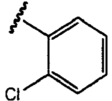 | 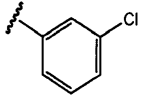 | 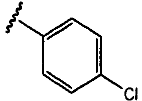 |
| Name: | 2-chlorophenyl - | 3-chlorophenyl | 4-chlorophenyl |
| Structure: | 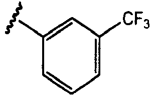 | 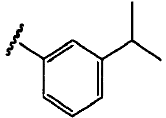 | 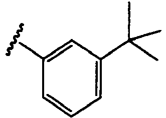 |
| Name: | 3-(trifluoromethyl)phenyl | 3-isopropylphenyl | 3-tert-butylphenyl |
| Structure: | 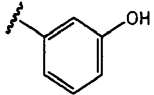 | 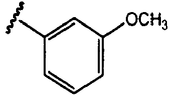 | 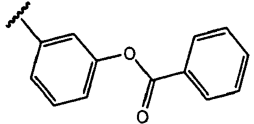 |
| Name: | 3-hydroxyphenyl | 3-methoxyphenyl | 3-(benzoyloxy)phenyl |
| **structure:** | 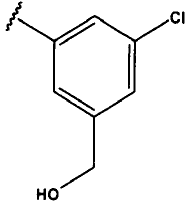 | 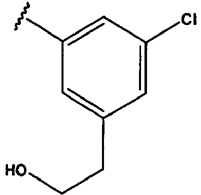 | 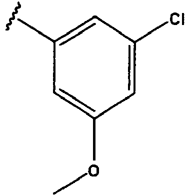 |
| **name:** | **3-chloro-5-(hydroximethyl)phenyl** | **3-chloro-5-(2-hydroxyethyl)phenyl** | **3-chloro-5-methoxyphenyl** |
| **structure:** | 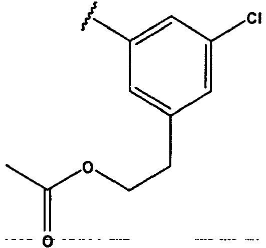 | | |
| **name:** | **3-(2-acetoxyethyl)-5-chlorophenyl** | | |
| Structure: | 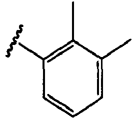 | 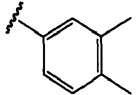 | 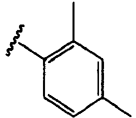 |
| Name: | 2,3-dimethylphenyl | 3.4-dimethylphenyl | 2,4-dimethylphenyl |
| Structure: | 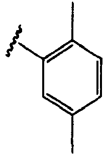 | 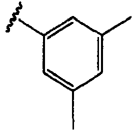 | 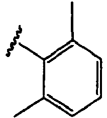 |
| Name: | 2,5-dimethylphenyl | 3,5-dimethylphenyl | 2,6-dimethylphenyl |
| Structure: | 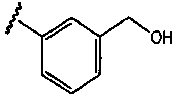 | 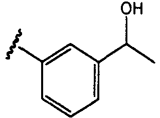 | 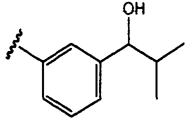 |
| Name: | 3-(hydroxymethyl)phenyl | 3-(1-hydroxyethyl)phenyl | 3-(1-hydroxy-2-methylpropyl)phenyl |
| Structure: | 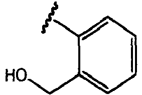 | 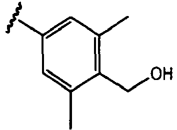 | 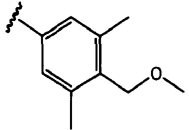 |
| Name: | 2-(hydroxymethyl)phenyl | 4-(hydroxymethyl)-3,5-dimethylphenyl | 4-(methoxymethyl)-3,5-dimethylphenyl |
| Structure: | 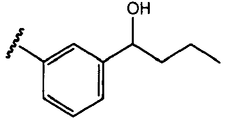 | 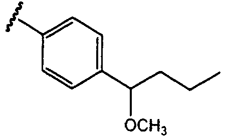 | 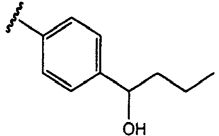 |
| Name: | 3-(1-hydroxybutyl)phenyl | 4-(1-methoxybutyl)phenyl | 4-(1-hydroxybutyl)phenyl |
| Structure: | 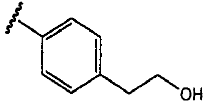 | 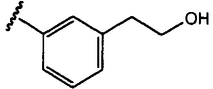 | 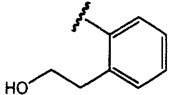 |
| Name: | 4-(2-hydroxyethyl)phenyl | 3-(2-hydroxyethyl)phenyl | 2-(2-hydroxyethyl)phenyl |
| Structure: | 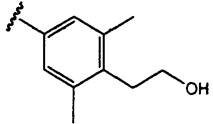 | 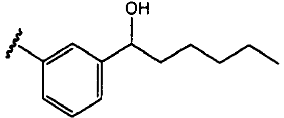 | 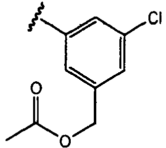 |
| Name: | 4-(2-hydroxylethyl)-3,5-dimethylphenyl | 3-(1-hydroxyhexyl)phenyl | 3-(acetoxymethyl)-5-chlorophenyl |
| Structure: | 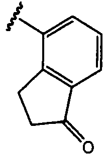 | 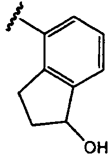 | 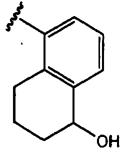 |
| Name: | 1-oxo-2,3-dihydro-1H-inden-4-yl | 1-hydroxy-2,3-dihydro-1H-inden-4-yl | 5-hydroxy-5,6,7,8-tetrahydronaphthalen-1-yl |
| Structure: | 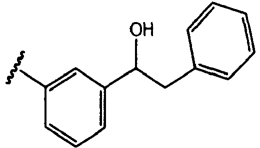 | 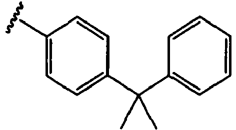 | |
| Name: | 3-(1-hydroxy-2-phenylethyl)phenyl | 4-(2-phenylpropan-2-yl)phenyl | |
| Structure: | 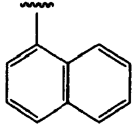 | 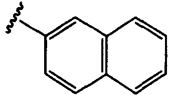 | |
| Name: | naphthalen-1-yl | naphthalen-2-yl | |
| Structure: | 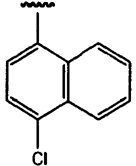 | | |
| Name: | 4-chloronaphthalen-1-yl | | |

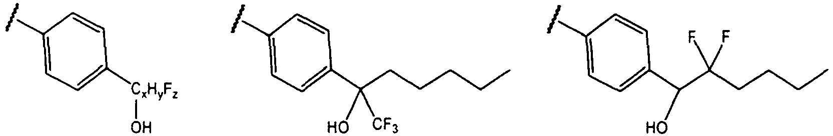

In the above embodiments, x is 5, 6, or 7, and y + z is 2x + 1.

In one embodiment, x is 5 and y + z is 11.

In another embodiment, x is 6 and y + z is 13.

In another embodiment, x is 7 and y + z is 15.

A compound, substituent, moiety, or any structural feature is stable if it is sufficiently stable for the compound to be isolated for at least 12 hours at room temperature under normal atmospheric conditions, or if it is sufficiently stable to be useful for at least one use disclosed herein.

The term aromatic refers to the meaning commonly understood in the art, i.e. it refers to an unsaturated, fully conjugated ring having 4N+2 ring electrons (e.g. 2, 6, 10, etc.) Thus, phenyl, pyridinyl, thienyl, furyl, and the like are aromatic. Aryl is a moiety that is aromatic.

A pharmaceutically acceptable salt is any salt th at retains the activity of the parent compound and does not impart any additional deleterious or untoward effects on the subject to which it is administered and in the context in which it is administered compared to the parent compound. A pharmaceutically acceptable salt also refers to any salt which may form in vivo as a result of administration of an acid, another salt, or a prodrug which is converted into an acid or salt Examples of useful salts include sodium salts, potassium salts, calcium salts, ammonium salts.

Unless otherwise indicated, reference to a compound should be construed broadly to include pharmaceutically acceptable salts of the depicted structure.

Unless stereochemistry is explicitly depicted, a structure is intended to include every possible stereoisomer, both pure or in any possible mixture. In particular, compounds having the stereochemistry indicated in the structures below are contemplated.

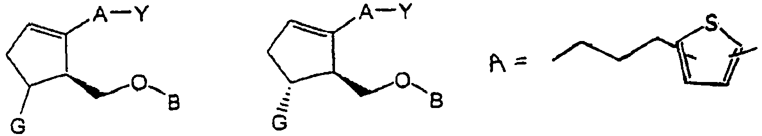

A person of ordinary skill in the art understands the meaning of the stereochemistry associated with the hatched wedge/solid wedge structural features. For example, an introductory organic chemistry textbook (Francis A. Carey, Organic Chemistry, New York: McGraw-Hill Book Company 1987, p. 63) states "a wedge indicates a bond coming from the plane of the paper toward the viewer" and the hatched wedge "represents a bond receding from the viewer."

For the purposes of this disclosure, "treat," "Treating." or "treatment" refer to the use of a compound. composition, therapeutically active agent, or drug in the diagnosis, cure, mitigation, treatment, prevention of disease or other undesirable condition.

Hypothetical useful compounds are depicted below,

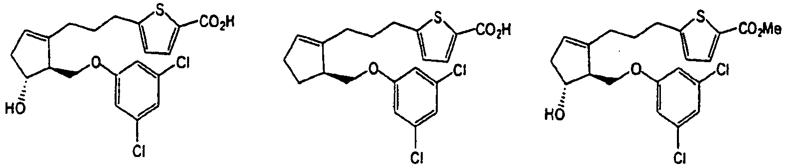

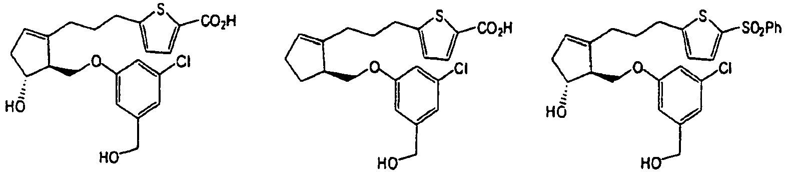

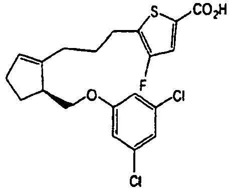

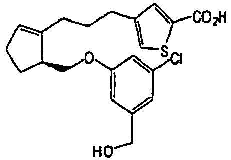

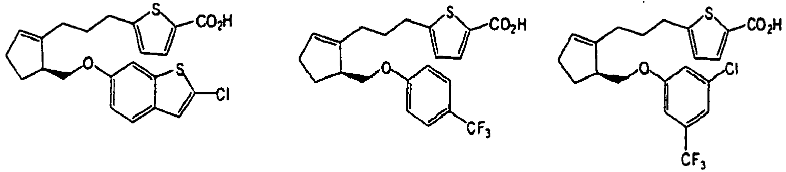

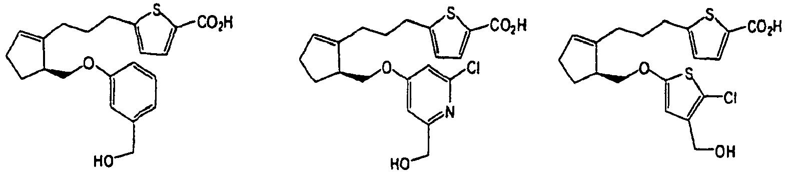

The compounds disclosed herein are useful in the treatment of glaucoma or ocular hypertension in a mammal.

Another embodiment is a medicament comprising a compound disclosed herein, wherein said composition is a liquid which is ophthalmically acceptable.

Another embodiment is a kit comprising a composition comprising a compound disclosed herein, a container, and instructions for administration of said composition to a mammal for treatment of glaucoma or ocular hypertension.

**Synthetic Methods**

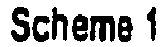

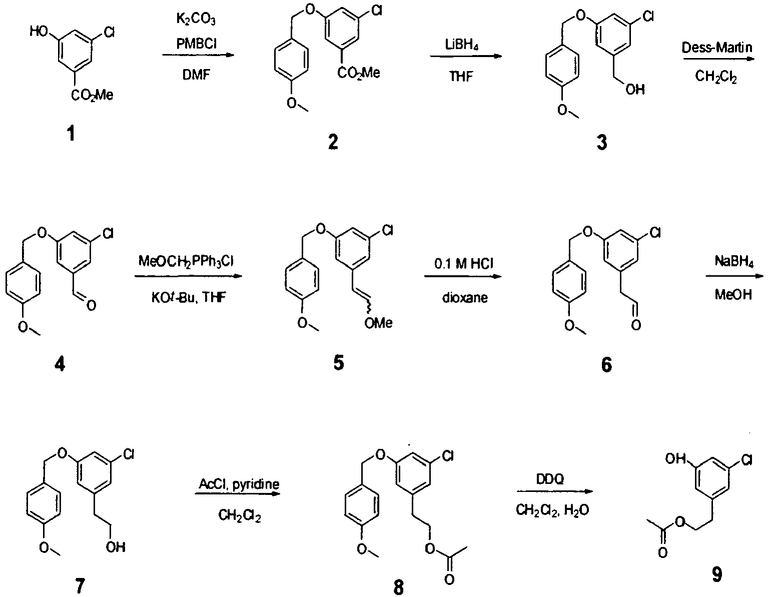

**Preparation 1**

3-chloro-5-hydroxyphenethyl acetate (**9,** Scheme 1)

Step 1. Protection of phenol **1** to give ether **2**

Potassium carbonate (4.3 g, 31.1 mmol) and 4-methoxybenzyl chloride (2.02 mL, 14.9 mmol) were added to a solution of phenol **1** (see US Provisional Patent Application No. 60/757,696, filed January 10, 2006, incorporated by reference herein, 2.30 g, 12.3 mmol) in DMF (100 mL). The mixture was heated at 100 °C. After 3 hours the mixture was allowed to cool to room temperature and then partitioned between water (150 mL) and EtOAc (200 mL). The phases were separated and the organic phase was washed with additional water (100 mL) and brine (50 mL). The organic phase was then dried (MgSO₄), filtered and concentrated in vacuo. Purification of the residue by flash column chromatography on silica gel (20% EtOAc/hexane) afforded 3.25 g (86%) of ether **2.**

Step 2. Reduction of **2** to give **3**

A solution of ester **2** (3.25 g, 10.6 mmol) in THF (17 mL) was added via syringe to a solution of LiBH₄ (0.346 g, 15.9 mmol) in THF (5 mL) at 0 °C. The mixture was heated at 80 °C overnight. The reaction mixture was allowed to cool to room temperature, quenched with water, diluted with 5% aqueous citric acid (100 mL) and extracted with EtOAc (75 mL). The organic phase was dried (MgSO₄), filtered and concentrated in vacuo. Purification of the crude residue by flash column chromatography on silica gel (30% EtOAc/hexane) afforded 2.91 g (99%) of alcohol **3.**

Step 3. Oxidation of **3** to give **4**

A solution of alcohol **3** (2.50 g, 8.97 mmol) in CH₂Cl₂ (125 mL) was added to a solution of Dess-Martin periodinane (4.57 g, 10.8 mmol) in CH₂Cl₂ (125 mL). After 2 hours at room temperature the reaction was partitioned between water (500 mL) and CH₂Cl₂ (300 mL). The phases were separated and the aqueous phase was extracted with CH₂Cl₂ (2 x 250 mL). The combined organic phase was washed with brine (200 mL) then dried (MgSO₄), filtered and concentrated in vacuo. Purification of the crude residue by flash column chromatography on silica gel (30% EtOAc/hexane) afforded 2.42 g (97%) of aldehyde **4.**

Step **4.** Wittig reaction of **4** to give **5**

Potassium tert-butoxide (2.54 g, 22.6 mmol) was added to a solution of methoxymethyltriphenylphosphonium chloride (3.72 g, 10.8 mmol) in THF (60 mL) at 0 °C. After 30 minutes at 0 °C, a solution of aldehyde **4** (2.5 g, 9.03 mmol) in THF (30 mL) was added. The reaction mixture was allowed to warm to room temperature and stirred **overnight.** The reaction was quenched at 0 °C by the slow addition of H₂O then was partitioned between 10% aqueous HCl (95 mL) and EtOAc (100 mL). The phases were separated and the aqueous phase was extracted with EtOAc (2 x 50 mL). The combined organic phase was washed with brine (20 mL) then dried (MgSO₄), filtered and concentrated in vacuo. Purification of the crude residue by flash column chromatography on silica gel (40% EtOAc/hexane) afforded 2.70 g (98%) of enol ether **5.**

Step 5. Hydrolysis of **5** to give **6**

M aqueous HCl (2.84 mL, 0.28 mmol) was added to a solution of enol ether **5** (2.70 g, 8.86 mmol) in dioxane (90 mL). After 1 hour at room temperature, the mixture was heated at 60 °C for 2.5 hours then cooled to room temperature. The reaction mixture was partitioned between saturated aqueous NaHCO₃ (300 mL) and CH₂Cl₂ (300 mL). The phases were separated and the aqueous phase was extracted with CH₂Cl₂ (2 x 300 mL). The combined organic phase was washed with H₂O and brine then dried (MgSO₄), filtered and concentrated in vacuo. Purification of the crude residue by flash column chromatography on silica gel (30% EtOAc/hexane) afforded 812 mg (32%) of aldehyde **6.**

Step 6. Reduction of **6** to give **7**

Sodium borohydride (159 mg, 4.20 mmol) was added to a solution of aldehyde **6** (812 mg, 2.79 mmol) in MeOH (34 mL) at 0 °C. The mixture was allowed to warm to room temperature. After 20 minutes at room temperature, the reaction was cooled to 0 °C and quenched by the slow addition of water. The mixture was then diluted with water (200 mL) and extracted with EtOAc (2 x 300 mL). The combined organic phase was washed with brine, dried (MgSO₄), filtered and concentrated in vacuo. Purification of the crude residue by flash column chromatography on silica gel (50% EtOAc/hexane) afforded 816 mg (99%) of alcohol **7.**

Step 7. Protection of **7** to give **8**

Pyridine (247 µL, 3.05 mmol) and acetyl chloride (216 µL, 3.04 mmol) were added sequentially to a solution of alcohol 7 (816 mg, 2.79 mmol) in CH₂Cl₂ (15 mL). After 5 min, the reaction mixture was partitioned between saturated aqueous NaHCO₃ (150 mL) and CH₂Cl₂ (150 mL). The phases were separated and the aqueous phase was extracted with CH₂Cl₂ (2 x 150 mL). The combined organic phases were washed with brine (150 mL), dried (mugSO₄), filtered and concentrated in vacuo. Purification of the crude residue by flash column chromatography on silica gel (10% EtOAc/hexane) afforded 850 mg (91%) of acetate **8.**

Step 8. Deprotection of **8** to give **9**

2,3-Dichloro-5,6-dicyano-1,4-benzoquinone (DDQ, 814 mg, 3.59 mmol) was added to a mixture of ether **8** (400 mg, 1.19 mmol) in CH₂Cl₂ (9 mL) and H₂O (0.45 mL) at 0 °C. After 1 hour at 0 °C the reaction was allowed to warm to room temperature. After 4 hours at room temperature, the reaction was quenched with saturated aqueous NaHCO₃ (100 mL). The mixture was extracted with CH₂Cl₂ (3x100 mL). The combined extracts were washed with water and brine then dried (MgSO₄), filtered and concentrated in vacuo. Purification of the residue by flash column chromatography on silica gel (30% EtOAdhexane) afforded 80 mg (31%) of the title compound (9).

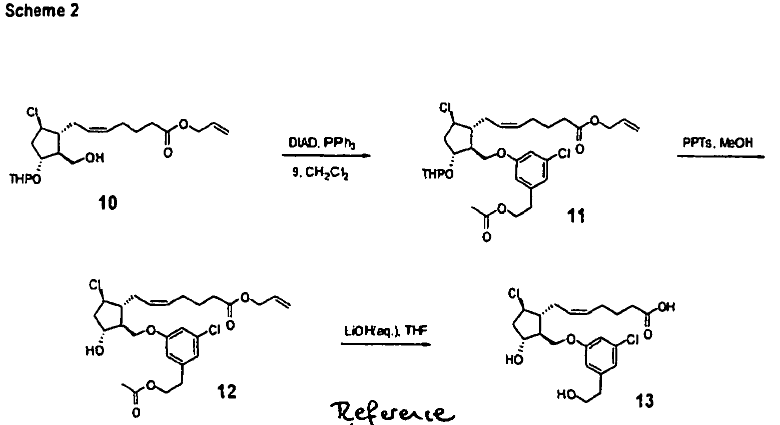

Reference Example 1

(Z)-7-{(1R,2S,3R.5R)-5-Chloro-2-[3-chloro-5-(2-hydroxyethyl)-phenoxymethyl[-3-hydroxy-cydopentylrhept-5-enoic acid (13, Scheme 2)

Step 1. Mitsunobu reaction of **9** and **10 to** give **11**

Triphenylphosphine (98 mg, 0.37 mmol) and diisopropyl azodicarboxylate (DIAD, 58 0L, 0.30 mmol) were added sequentially to a solution of alcohol 10 (see US Provisional Patent Application No. 60/757,696, filed January 10, 2006: 100 mg, 0.25 mmol) and phenol **9** (preparation 1, 80 mg, 0.37 mmol) in CH₂Cl₂ (1.0 mL). After stirring 18 hours at room temperature, the reaction mixture was partitioned between saturated aqueous NaHCO₃) (20 mL) and CH₂Cl₂ (15 mL). The phases were separated and the aqueous phase was extracted with CH₂Cl₂ (2 x 20 mL). The combined organic phase was washed with brine (15 mL), dried (MgSO₄), filtered and concentrated in vacuo. Purification of the residue by flash column chromatography on silica gel (20% EtOAc/hexane) afforded 108 mg (72%) of aryl ether **11.**

Step 2: Deprotection of **11 to** give **12.**

Pyridinium p-toluenesulfonate (PPTs, 4.7 mg, 0.019 mmol) was added to a solution of **11** (108 mg, 0.18 mmol) in methanol (2.0 mL) at room temperature under nitrogen. The solution was heated at 40 °C for 5 h, then cooled and concentrated in vacuo. Purification of the crude residue by flash column chromatography on silica gel (50% EtOAc/hexane) afforded 53 mg (57%) of alcohol **12.**

Step 3: Hydrolysis of **12** to give **13**

Lithium hydroxide (0.15 mL of a 1.0 M aqueous solution, 0.15 mmol) was added to a solution of ester 12 (13 mg, 0.025 mmol) in THF (0,13 mL). After 2 hours room temperature, the reaction was partitioned between 10% aqueous HCl (3 mL) and EtOAc (7 mL). The phases were separated and the aqueous phase was extracted with EtOAc (2 x 7 mL). The combined organic phase was washed with brine, dried (MgSO₄), filtered and concentrated in vacuo to afford 11 mg (quant.) of the title compound **(13)**.

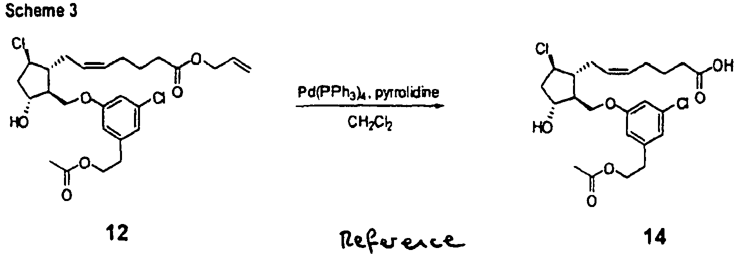

Reference Example 2

(Z)-7-((1R,2S,3R,5R)-2-[3-(2-Acetoxy-ethyl)-5-chloro-phenoxymethyl]-5-chloro-3-hydroxy-cyclopentyl)-hept-5-enoic acid (14, Scheme 3)

Tetrakis(triphenylphosphine)palladium(0) (20 mg, 0.017 mmol) and pyrrolidine (14 0L. 0.17 mmol) were added sequentially to a solution of allyl ester **12** (30 mg, 0.058 mmol) in CH₂Cl₂ (1.0 mL). After 5 min the reaction mixture was partitioned between 1.0 M aqueous HCl (5 mL) and CH₂Cl₂ (15 mL). The phases were separated and the aqueous phase was extracted with CH₂Cl₂ (2 x 10 mL). The combined extracts were washed with brine (10 mL), dried (MgSO₄), filtered and concentrated in vacuo. Purification of the crude residue by flash column chromatography on silica gel (60% EtOActhexane) afforded 9 mg (33%) of the title compound (**14**).

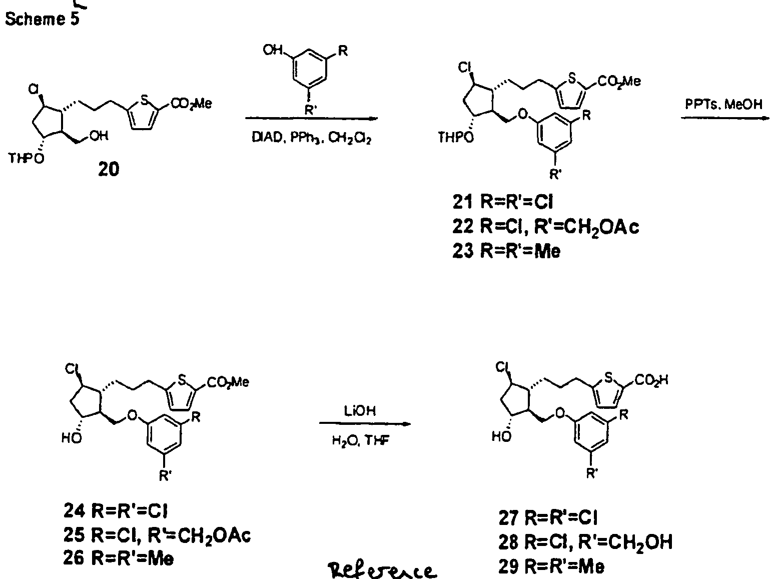

Reference Example 3

5-{3-[(1R,2S,3R,5R)-5-Chloro-2-(3,5-dichloro-phenoxymethyl)-3-hydroxy-cyclopentyl)-propyl}-thiophene-2-carboxylic acid (27, Scheme 5)

Step 1. Mitsunobu reaction of 20 to give 21

Triphenylphosphine (38 mg, 0.14 mmol) and DIAD (23 µL, 0.12 mmol) were added to a solution of alcohol 20 (see US Provisional Patent Application No. 60/805,285, filed June 20, 2006, incorporated by reference herein; 40 mg, 0.096 mmol) and 3,5-dichlorophenol (23 mg, 0.14 mmol) in CH₂Cl₂ (1.0 mL). After stirring 18 hours at room temperature, the mixture was partitioned between CH₂Cl₂ (10 mL) and saturated aqueous NaHCO₃ (10 mL). The phases were separated and the aqueous phase was extracted with CH₂Cl₂ (2 x 10mL). The combined organic phase was washed with brine (10 mL), dried (MgSO₄), filtered and concentrated in vacuo. Purification of the residue by flash column chromatography on silica gel (hexane → EtOAc, gradient) afforded **20** mg (37%) of **21**.

Step 2. Deprotection of **21** to give **24**

Pyridinium p-toluenesulfonate (PPTs, 1 mg, 0.004 mmol) was added to a solution of **21** (20 mg, 0.036 mmol) in methanol (0.35 mL) at room temperature. The solution was heated at 40 °C overnight then cooled and concentrated in vacuo. Purification of the crude residue by flash column chromatography on silica gel (hexane → EtOAc, gradient) afforded 10 mg (59%) of **24.**

Step 3. Hydrolysis of **24** to give **27**

Ester **24** (10mg, 0.021 mmol) was converted into 3 mg (31%) of the title compound (**27**) in accordance with the procedure of Reference Example 1, step 3 with the following modifications: the reaction was stirred for 18 hours at room temperature, and the crude product was purified by flash column chromatography on silica gel (10% MeOH/CH₂Cl₂).

Reference Example 4

5-{3-((1R,2S,3R,SR)-5-Chloro-2-(3,5-dimethyl-phenoxymethyl)-3-hydroxy-cydopentyl]-propyl}-thiophene-2-carboxylic acid (29, Scheme 5)

Step 1. Mitsunobu reaction of **20** to give **23**

Triphenylphosphine (47 mg, 0.18 mmol) and DIAD (27µL, 0.14 mmol) were added to a solution of alcohol **20** (see US Provisional Patent Application No. 60/805,285, filed June 20, 2006; 50 mg, 0.12 mmol) and 3,5-dimethylphenol (17 mg, 0.14 mmol) in CH₂Cl₂ (0.6 mL). After stirring 18 hours at room temperature, the mixture was partitioned between CH₂Cl₂ (10 mL) and saturated aqueous NaHCO₃ (10 mL). The phases were separated and the aqueous phase was extracted with CH₂Cl₂ (2 x 10 mL). The combined organic phase was washed with brine (10 mL), dried (MgSO₄), filtered and concentrated in vacuo. Purification of the residue by flash column chromatography on silica gel (hexane → EtOAc, gradient) afforded 53 mg (85%) of **23.**

Step 2. Deprotection of **23** to give **26**

Acetal **23** (53 mg, 0.10 mmol) was converted into **37** mg (83%) of alcohol **26** in accordance with the procedure of Reference Example 3, step 2

Step 3. Hydrolysis of **26** to give **29**

Ester 26 (37 mg, 0.085 mmol) was converted into 15 mg (42%) of the title compound (29) in accordance with the procedure of Reference Example 1, step 3 with the following modifications: the reaction was stirred for 18 hours at 40 °C, and the crude product was purified by flash column chromatography on silica gel (10% MeOH/CH₂Cl₂).

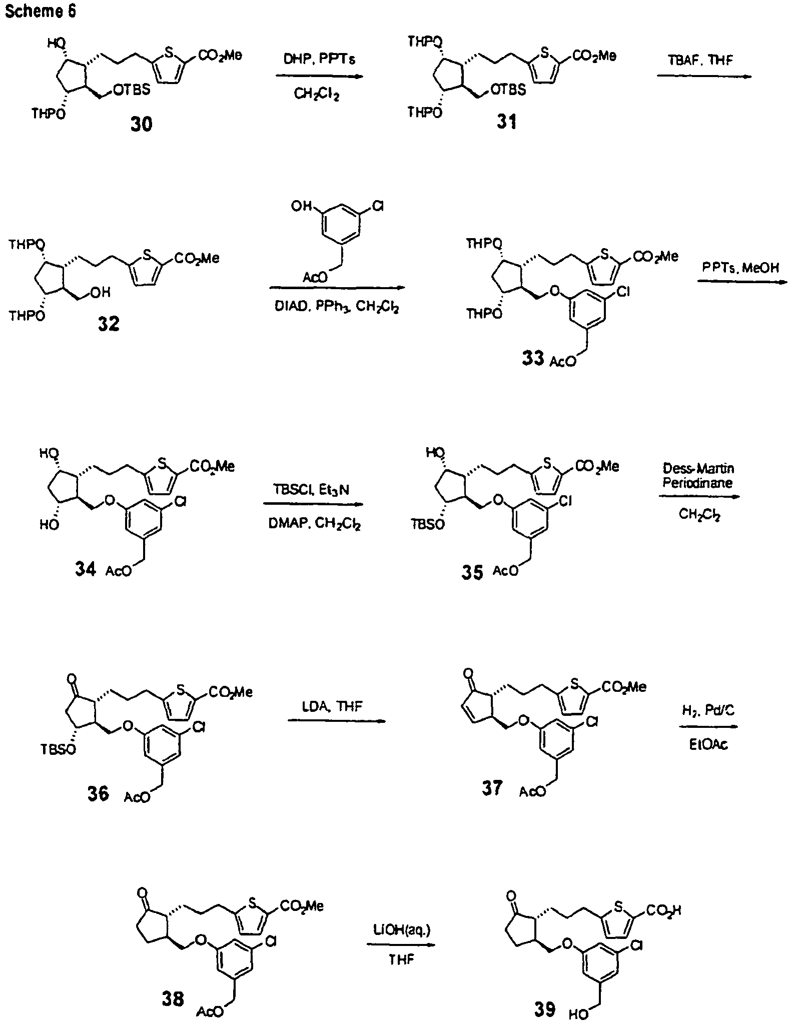

Reference Example 5

5-(3-[(1R.2S)-2-(3-Chloro-5-hydroxymethyl-phenoxymethyl)-5-oxo-cyclopentyl)-propyl)-thiophene-2-carboxylic acid (39, Scheme 6)

Step 1. Protection of **30** to give **31**

Dihydropyran (391 µL, 4.29 mmol) and PPTs (50 mg, 0.20 mmol) were added to a solution of alcohol **30** (see US Provisional Patent Application No. 60/805,285, filed June 20, 2006; 550 mg, 1.07 mmol) in CH₂Cl₂ (3.0 mL). The reaction mixture was heated at 40 °C overnight, then cooled and concentrated in vacuo. Purification of the crude residue by flash column chromatography on silica gel (hexane → EtOAc, gradient) afforded 550 mg (86%) of **31.**

Step 2. Desilylation of **31** to give **32**

Tetrabutylammonium fluoride (2.51 mL of a 1.0 M THF solution, 2.51 mmol) was added to a solution of **31** (500 mg, 0.84 mmol) in THF (7.6 mL). After 18 hours at room temperature, the reaction mixture was partitioned between water (10mL) and EtOAc (20 mL). The phases were separated and the aqueous phase was extracted with EtOAc (2 x 10 mL). The combined extracts were washed with brine then dried (MgSO₄), filtered and concentrated in vacuo. Purification of the crude residue by flash column chromatography on silica gel (hexane → EtOAc, gradient) afforded 393 mg (97%) of **32.**

Step 3. Mitsunobu of **32** to give **33**

Alcohol **32** (437 mg, 0.91 mmol) and 3-chloro-5-hydroxybenzyl acetate (see US Provisional Patent Application No. 60/757,696, filed January 10, 2006; 218 mg, 1.09 mmol) were converted into 350 mg (58%) of aryl ether **33** in accordance with the procedure of Reference Example 4, step 1.

Step 4. Deprotection of **33** to give **34**

Bis-acetal 33 (350 mg, 0.53 mmol) was converted into **150** mg (57%) of diol **34** in accordance with the procedure of Reference Example 3, step 2.

Step 5. Monosilylation of **34** to give **35**

Triethylamine (63 0L, 0.45 mmol), dimethylaminopyridine (7 mg, 0.057 mmol), and tert-butyldimethylsilyl chloride (50 mg, 0.33 mmol) were sequentially added to a solution of **34** (150 mg, 0.30 mmol) in CH₂Cl₂ (1.5 mL). After stirring 18 hours at room temperature, the mixture was partitioned between CH₂Cl₂ (10 mL) and saturated aqueous NaHCO₃ (5 mL). The phases were separated and the aqueous phase was extracted with CH₂Cl₂ (2 x 10 mL). The combined organic phase was washed with brine (10 mL), dried (MgSO₄), filtered and concentrated in vacuo. Purification of the residue by flash column chromatography on silica gel (hexane → EtOAc, gradient) afforded **90** mg (49%) of **35.**

Step 6. Oxidation of **35** to give **36**

Dess-Martin periodinane (75 mg, 0.18 mmol) was added to a solution of **35** (90 mg. 0.15 mmol) in CH₂Cl₂ (7.35 mL) at 0 °C and the mixture was allowed to warm to room temperature. After 2 hours at room temperature, the mixture was partitioned between CH₂Cl₂ (10 mL) and water (10 mL). The phases were separated and the aqueous phase was extracted with CH₂Cl₂ (2 x 10 mL). The combined organic phase was washed with brine (5 mL), dried (MgSO₄), filtered and concentrated in vacuo. Purification of the residue by flash column chromatography on silica gel (hexane → EtOAc, gradient) afforded 80 mg (89%) of ketone **36.**

Step 7. Elimination of **36** to give **37**

A solution of lithium diisopropylamide (0.41 mL of a 2.0 M solution in heptane-THF-ethylbenzene, 0.82 mmol) was added to a solution of 36 (80 mg, 0.13 mmol) in THF (2.3 mL) at - 78°C. After 90 minutes at - 78 °C, the mixture was allowed to warm to room temperature. After 15 minutes at room temperature, the reaction was quenched by the addition of 0.1 N aqueous HCl (15 mL), and extracted with EtOAc (3 x 20 mL). The combined extracts were washed with brine, dried (MgSO₄), filtered and concentrated in vacuo. Purification of the residue by flash column chromatography on silica gel (hexane → EtOAc, gradient) afforded 40 mg (64%) of enone **37.**

Step 8. Hydrogenation of **37** to give **38**

Palladium on carbon (10 wit.%. 8 mg) was added to a solution of enone **37** (40 mg, 0.084 mmol) in EtOAc (1.6 mL). A hydrogen atmosphere was established by evacuating and refilling with hydrogen (5x) and the reaction mixture was stirred under a balloon of hydrogen for 18 hours. The reaction mixture was filtered through celite, washing with EtOAc, and the filtrate was concentrated in vacuo to afford 31 mg (77%) of saturated ketone **38.**

Step 9. Hydrolysis of **38** to give **39**

Ester **38** (5 mg, 0.010 mmol) was converted into 3.5 mg (79%) of the title compound **(39)** in accordance with the procedure of Reference 3 example 3, step 3.

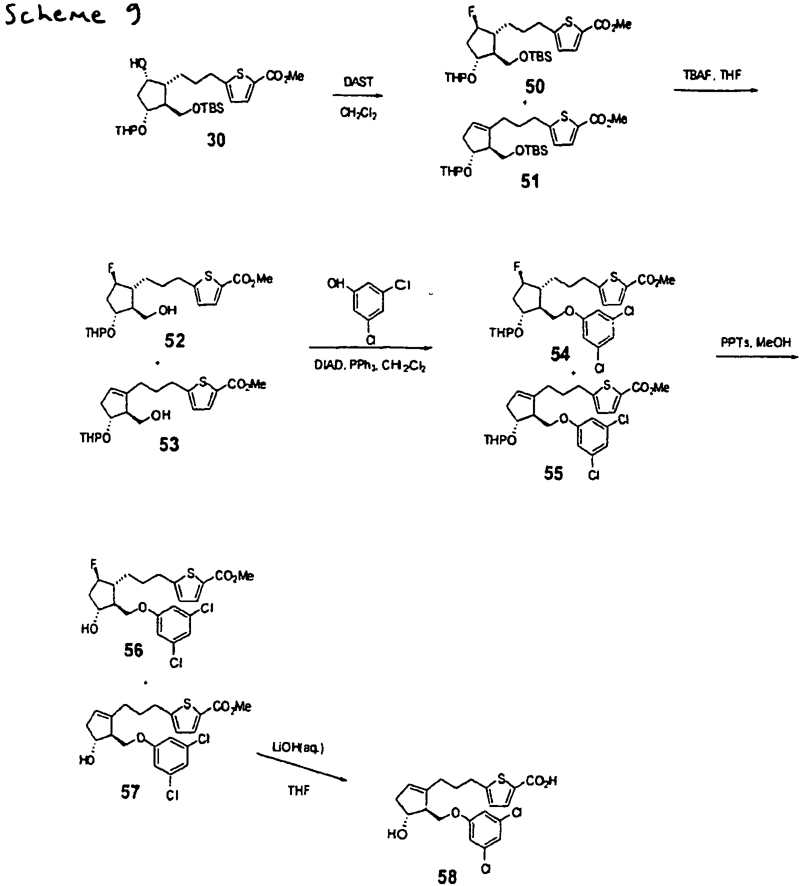

**Exampl**e 1

5-{3[(4R,5S)-5-(3,5-Dichloro-phenoxymethyl)-4-hydroxy-cyclopent·1-enyl]-propyl}-thiophene-2-carboxylic acid (**58**, Scheme 9)

Step 1. Conversion of 30 to fluoride **50** and alkene **51**

(Diethylamino)sulfur trifluoride (DAST, 104 µL, 0.79 mmol) was added to a solution of alcohol 30 (see US Provisional Patent Application No. 60/805,285, filed June 20, 2006; 200 mg, 0.39 mmol) in CH₂Cl₂ (92 mL) at - 78 °C. After 30 minutes at room temperature, the reaction was quenched with saturated aqueous NaHCO₃ (25 mL). The mixture was diluted with water (25 mL) and extracted with CH₂Cl₂ (2 x 25 mL). The combined organic phase was dried (MgSO₄), filtered and concentrated in vacuo. Purification of the residue by flash column chromatography on silica gel (hexane → EtOAc, gradient) afforded 42 mg (-20%) of an inseparable mixture of **50** and **51**.

Step 2. Disilylation of **50/51** to **52/53**

Silyl ethers **50/51** (42 mg, -0.08 mmol) were converted into 25 mg (-77%) of inseparable alcohols **52/53** in accordance with the procedure Reference Example 5 step 2.

Step 3. Mitsunobu of 52/53 to 54/55

Alcohols **52/53** (25 mg, -0.06 mmol) and 3,5-dichlorophenol (9 mg 0.055 mmol) were converted into 24 mg (-70%) of inseparable aryl ethers **54/55** in accordance with the procedure of Reference Example 4, step 1.

Step 4. Deprotection of **54/55** to **56** and **57**

Acetals **54/55** (24 mg, -0.45 mmol) were converted into 1 mg (-5%) of hydroxyl alkene **57** and 20 mg (-83%) of a mixture of **56** and **57** in accordance with the procedure of Reference Example 3, step 2.

Step 5. Hydrolysis of **57** to **58**

Ester 57 (1mg. 0.022 mmol) was converted into 1 mg (ouant) of the title compound (**58**) in accordance with the procedure of Reference Example 4. step 3.

**In vitro testing**

United States Patent Application Serial No. 11/553,143, filed on October 26, 2006, describes the methods used to obtain the in vitro data in the table below.

| Structure | hEP2 | | | hEP4 | | Other Receptors | | | | | |
|---|---|---|---|---|---|---|---|---|---|---|---|
| | flipr EC50 | cAMP EC50 | Kl | flipr EC50 | Ki | hFP | hEP1 | hEP3 | hTP | hlP | hDP |
| 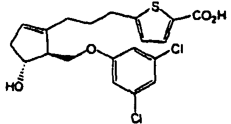 | 424 | 4.8 | 81 | >10000 | 1584 | NA | NA | NA | NA | NA | NA |

## Claims

1. A compound represented by the following formula or a pharmaceutically-acceptable salt thereof: 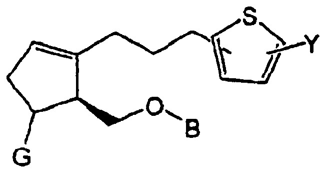 wherein:
Y is an organic acid functional group, an amide or ester of an organic acid functional group comprising up to 14 carbon atoms, hydroxymethyl, an ether of hydroxymethyl comprising up to 14 carbon atoms, or a tetrazolyl functional group;
G is H or OH; and
B is an optionally-substituted aryl group or an optionally-substituted heteroaryl group, the total number of non-hydrogen atoms in the optional substituents being 20 or less.

2. A compound according to Claim 1, wherein the compound is represented by the following formula or is a pharmaceutically-acceptable salt thereof: 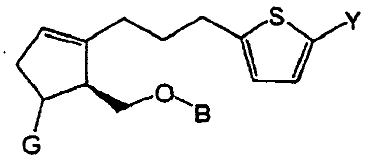 wherein Y, G and B are as defined in Claim 1.

3. A compound according to Claim 1 or Claim 2, wherein B is an optionally-substituted phenyl group.

4. A compound according to Claim 3, wherein B is 3-chloro-5-(hydroxymethyl)phenyl.

5. A compound according to Claim 3, wherein B is 3-chloro-5-(2-hydroxyethyl)phenyl.

6. A compound according to Claim 3, wherein B is 3-chloro-5-methoxyphenyl.

7. A compound according to Claim 3, wherein B is 3-(2-acetoxyethyl)-5-chlorophenyl.

8. A compound according to any preceding claim, wherein G is H.

9. A compound according to any of Claims 1 to 7, wherein G is OH.

10. A compound according to any preceding claim, wherein Y is CO₂R², CON(R²)₂, CON (OR²) R², CON (CH₂CH₂OH)₂, CONH (CH₂CH₂OH), CH₂OH, P(O)(OH)₂, CONHSO₂R², SO₂N (R²) ₂, SO₂NHR², 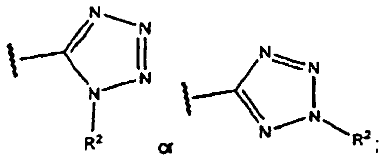 wherein R² is H, C₁-C₆ alkyl, unsubstituted phenyl or unsubstituted biphenyl.

11. A compound according to Claim 10, wherein Y is CO₂R².

12. A composition comprising a compound according to any preceding claim, the composition being an ophthalmically-acceptable liquid.

13. A compound according to any of Claims 1-11 for use in the treatment of glaucoma or ocular hypertension in a mammal.

## Patentansprüche

1. Verbindung der nachstehenden Formel oder ein pharmazeutisch annehmbares Salz davon: 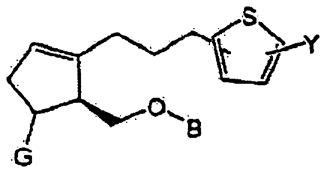 worin:
Y eine organische funktionelle Säuregruppe, ein Amid oder Ester einer organischen funktionellen Säuregruppe mit bis zu 14 Kohlenstoffatomen, Hydroxymethyl, ein Ether von Hydroxymethyl mit bis zu 14 Kohlenstoffatomen oder eine Tetrazolylgruppe ist;
G H oder OH ist; und
B eine gegebenenfalls substituierte Arylgruppe oder eine gegebenenfalls substituierte Heteroarylgruppe ist, wobei die Gesamtzahl an Nicht-Wasserstoffatomen in den optionalen Substituenten 20 oder weniger ist.

2. Verbindung gemäss Anspruch 1, wobei die Verbindung durch die nachstehende Formel oder ein pharmazeutisch annehmbares Salz davon dargestellt ist: 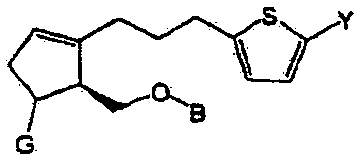 worin Y, G und B wie in Anspruch 1 definiert sind.

3. Verbindung gemäss Anspruch 1 oder Anspruch 2, worin B eine gegebenenfalls substituierte Phenylgruppe ist.

4. Verbindung gemäss Anspruch 3, worin B 3-Chlor-5-(hydroxymethyl)phenyl ist.

5. Verbindung gemäss Anspruch 3, worin B 3-Chlor-5-(2-hydroxyethyl) phenyl ist.

6. Verbindung gemäss Anspruch 3, worin B 3-Chlor-5-methoxyphenyl ist.

7. Verbindung gemäss Anspruch 3, worin B 3-(2-Acetoxyethyl) -5-chlorphenyl ist.

8. Verbindung gemäss irgendeinem der vorhergehenden Ansprüche, worin G H ist.

9. Verbindung gemäss irgendeinem der Ansprüche 1 bis 7, worin G OH ist.

10. Verbindung gemäss irgendeinem der vorhergehenden Ansprüche, worin Y CO₂R², CON(R²)₂, CON(OR²)R², CON(CH₂CH₂OH)₂, CONH(CH₂CH₂OH), CH₂OH, P(O)(OH)₂, CONHSO₂R², SO₂N(R²)₂, SO₂NHR², 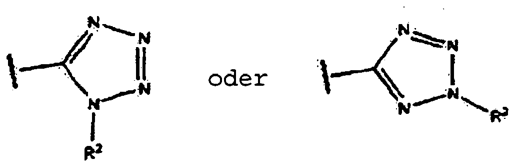 ist, worin R² H, C₁₋₆-Alkyl, unsubstituiertes Phenyl oder unsubstituiertes Biphenyl ist.

11. Verbindung gemäss Anspruch 10, worin Y CO₂R² ist.

12. Zusammensetzung, umfassend eine Verbindung gemäss irgendeinem der vorhergehenden Ansprüche, wobei die Zusammensetzung eine ophthalmisch annehmbare Flüssigkeit ist.

13. Zusammensetzung gemäss irgendeinem der Ansprüche 1 bis 11 zur Verwendung bei der Behandlung von Glaukom oder erhöhtem Augeninnendruck in einem Säuger.

## Revendications

1. Composé représenté par la formule suivante ou un sel pharmaceutiquement acceptable de celui-ci : 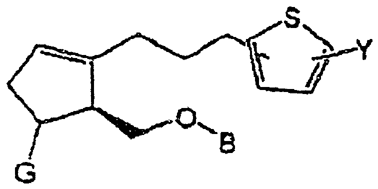 dans laquelle :
Y représente un groupe fonctionnel acide organique, un amide ou un ester d'un groupe fonctionnel acide organique comprenant jusqu'à 14 atomes de carbone, un hydroxyméthyle, un éther d'hydroxyméthyle comprenant jusqu'à 14 atomes de carbone, ou un groupe fonctionnel tétrazolyle ;
G représente H ou OH ; et
B représente un groupe aryle éventuellement substitué ou groupe hétéroaryle éventuellement substitué, le nombre total d'atomes autres que l'hydrogène dans les substituants éventuels étant inférieur ou égal à 20.

2. Composé selon la revendication 1, dans lequel le composé est représenté par la formule suivante ou par un sel pharmaceutiquement acceptable de celui-ci : 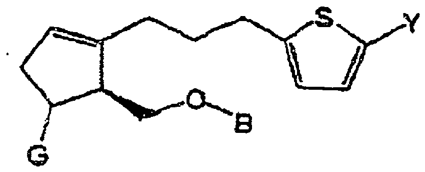 dans lequel Y, G et B sont tels que définis dans la revendication 1.

3. Composé selon la revendication 1 ou la revendication 2, dans lequel B représente un groupe phényle éventuellement substitué.

4. Composé selon la revendication 3, dans lequel B représente un 3-chloro-5-(hydroxyméthyl)phényle.

5. Composé selon la revendication 3, dans lequel B représente un 3-chloro-5-(2-hydroxyéthyl)phényle.

6. Composé selon la revendication 3, dans lequel B représente un 3-chloro-5-méthoxyphényle.

7. Composé selon la revendication 3, dans lequel B représente un 3-(2-acétoxyéthyl)-5-chlorophényle.

8. Composé selon l'une quelconque des revendications précédentes, dans lequel G représente H.

9. Composé selon l'une quelconque des revendications 1 à 7, dans lequel G représente OH.

10. Composé selon l'une quelconque des revendications précédentes, dans lequel Y représente CO₂R², CON(R²)₂, CON(OR²)R², CON(CH₂CH₂OH)₂, CONH(CH₂CH₂OH), CH₂OH, P(O)(OH)₂, CONHSO₂R², SO₂N(R²)₂, SO₂NHR², 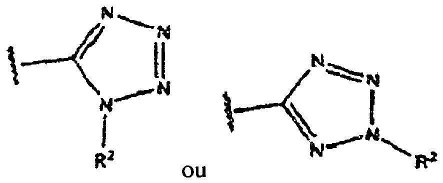 dans lequel R² représente H, un alkyle en C₁ à C₆, un phényle non substitué ou un biphényle non substitué.

11. Composé selon la revendication 10, dans lequel Y représente CO₂R².

12. Composition comprenant un composé selon l'une quelconque des revendications précédentes, la composition étant un liquide acceptable du point de vue ophtalmologique.

13. Composé selon l'une quelconque des revendications 1 à 11, destiné à être utilisé dans le traitement d'un glaucome ou de l'hypertension oculaire chez un mammifère.
